# EUROPEAN PATENT SPECIFICATION

(11) **EP 3 646 784 B1**
(45) Date of publication and mention of the grant of the patent: **16.12.2020**
(21) Application number: 18020575.9
(22) Date of filing: 05.11.2018
(51) Int. Cl.: A61B 5/048, A61B 5/0484, A61B 5/16

(54) **ELECTROENCEPHALOGRAPHIC METHOD AND APPARATUS FOR MEASURING SENSORY STIMULATION SALIENCE**
ELEKTROENZEPHALOGRAPHISCHES VERFAHREN UND VORRICHTUNG ZUR MESSUNG DER SENSORISCHEN STIMULATIONSSALIENZ
PROCÉDÉ D'ÉLECTROENCÉPHALOGRAPHIE ET APPAREIL DE MESURE DE SAILLANCE DE STIMULATION SENSORIELLE

(43) Date of publication of application: 06.05.2020
(73) Proprietor: Termobit Prod SRL, 020281 Bucharest (RO)
(72) Inventor: Moldovan, Mihai, Bucharest (RO); Serban, Cosmin-Andrei, Bucharest (RO); Barborica, Andrei, Bucharest (RO); Roceanu, Adina-Maria, Bucharest (RO); Mindruta, Ioana-Raluca, Voluntari City, Ilfov (RO); Zagrean, Ana-Maria, Bucharest (RO); Zagrean, Leon, Bucharest (RO); Ciurea, Jan, Bucharest (RO)
(74) Representative: Cosmovici, Paul

(56) References cited:
- RO-A- 132 025
- TOMAS ROS ET AL: "Mind over chatter: Plastic up-regulation of the fMRI salience network directly after EEG neurofeedback", NEUROIMAGE, vol. 65, 26 September 2012 (2012-09-26), pages 324-335, XP055578181, AMSTERDAM, NL ISSN: 1053-8119, DOI: 10.1016/j.neuroimage.2012.09.046
- ZHEN LIANG ET AL: "Characterization of electroencephalography signals for estimating saliency features in videos", NEURAL NETWORKS., vol. 105, 12 May 2018 (2018-05-12), pages 52-64, XP055582430, GB ISSN: 0893-6080, DOI: 10.1016/j.neunet.2018.04.013

## Description

### BACKGROUND OF THE INVENTION

The present invention relates to a method and an apparatus for determining a salient reactivity of at least one target sensory stimulus applied to a subject.

In its broadest sense, salience (also called saliency) of a sensory stimulus refers to its ability to stand-out and direct (grab) attention. Salience detection is considered to be a key attentional mechanism that facilitates learning and survival by focusing on the most relevant sensory data.

Salience is mostly studied in vision, although the concepts could be generalized to other sensory modalities. Attentional redirection towards a visually-salient stimulus can be measured by eye-tracking technologies. Such behavioral measurements can give information about the perceptual ("physical") salience of a sensory stimulus which typically arises from contrasts between items (targets) and their neighbors such as a red dot surrounded by white dots, although it is also likely that some of the physical features of the stimuli have evolved as special behavioral signals, like seeing the red color (loan et al. , 2007, Ilie et al. , 2008).

Perceptual salience reaches awareness rapidly and effortlessly. Nevertheless, perceptual salience detection is in competition with slower memory-dependent, or anticipatory mechanisms, such as when looking ahead of moving objects before crossing streets, referred to as attentional salience. Moreover, such attentional salience integrates perceptual salience - if something is perceptually salient, it is likely to draw attention as well. Thus, from the point of view of salience measurements, it is most advantageous to assess the slow attentional salience mechanisms, referred to as "top-down", although this cannot be addressed in behavioral measures.

Most neurophysiological studies investigating salience are carried out by EEG event related potentials, and subsequent event-related synchronization/desynchronization measures in the frequency domain where the salient target stimuli are presented in oddball designs ( 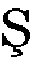 erban et al. , 2017). As such, they are inherently focused on the fast-perceptual aspects of salience under the influence of novelty and expectancy confounders and provide only limited information on the slow attentional salience networks.

Brain network studies by blood oxygen level-dependent functional magnetic resonance imaging MRI provided evidence for a baseline of neuronal ongoing activity, from which specific task-related transient changes, generally named activations, arise. Such "spontaneous activity" is organized in multiple highly specific functional anatomical networks referred to as resting state networks (RSNs), which are largely comprised of a large default mode network (DMN) including the posterior cingulate cortex , putatively associated with "mind-wandering" and internal processing, a "salience network" (SN) anchored by dorsal anterior cingulate and orbital fronto-insular cortices with robust connectivity to subcortical and limbic structures, and an "executive-control network" (ECN) that links dorsolateral frontal and parietal neocortices.

The activity within RSNs oscillates with frequencies between 0.01 and 0.1 Hz. In contrast, the oscillatory electrical activity of the brain recorded by EEG occurs in faster frequency bands broadly characterized as delta (1-4 Hz), theta (4-8 Hz), alpha (8-12 Hz) and beta (12-30 Hz). It thus became apparent that rather than reflecting a single EEG frequency, the EEG correlates of RSN activities represent a combination of different EEG rhythms, referred to as their "EEG signature" in the frequency domain (Mantini et al. , 2007). This approach identified that the SN is primarily related to activity in the alpha and beta bands, although this was similar to those observed in the DMN.

Previous studies have found that, in the voltage-domain, the scalp voltage topography remains quasi-stable for periods of about 80-120 ms and that an alternating sequence of only 4 such "microstates" (termed A-D) can account for most of the EEG variance. By convoluting the time-course of the microstate sequences with the hemodynamic response function, a correlation was found between microstate D (mostly frontal and medial) and the SN, however, no correlation was found between any microstates and DMN or between microstate occurrence and the EEG rhythms (Britz et al., 2010). In Tomas Ros et al: "Mind over chatter: Plastic up-regulation of the fMRI salience network directly after EEG neurofeedback", NEUROIMAGE, vol. 65, 26 September 2012 (2012-09-26), pages 324-335, salient reactivity is determined based on EEG obtained during sensory stimulation.

Although there is a growing interest to measure salience in neurology, psychiatry, social sciences and neuroscience, there is currently no satisfactory method to measure salience.

### SUMMARY OF THE INVENTION

The present invention relates to computer-implemented method according to independent claim 1, to an apparatus according to independent claim 11, and to a computer-readable storage medium according to independent claim 12. Preferred embodiments are described in the dependent claims 2 to 10.

A first aspect of the present invention provides a method for determining a salient reactivity of at least one target sensory stimulus applied to a subject, the method comprising:
- obtaining physiological signals from the subject during a stimulation with the target sensory stimulus and during a stimulation with a reference sensory stimulus, the physiological signals comprising a plurality of channels, wherein the physiological signals are EEG signals,
- determining, at a plurality of time points of the physiological signals, topographic spectral power maps, wherein a topographic spectral power map comprises spectral powers of frequency bands of the plurality of channels,
- clustering the topographic spectral power maps to obtain a plurality of macrostates,
- identifying, among the plurality of macrostates, a default macrostate as the macrostate which shows a largest suppression during stimulation, wherein a suppression of a macrostate during a sensory stimulus is determined as a difference between the probability of occurrence of the macrostate during the sensory stimulus compared to the probability of occurrence of the macrostate in a period before the sensory stimulus, and
- determining the salient reactivity based on a difference between the suppression of the default macrostate during the target sensory stimulus and a suppression of the default macrostate during the reference sensory stimulus.

The plurality of channels may correspond to a plurality of locations of the EEG electrodes. Preferably, the method is carried out over a plurality of trials, wherein further preferably the kind of stimulus may be varied over the plurality of trials (e.g. alternating between different kinds of sensory stimulation).

Obtaining the physiological signals may be performed by measuring the physiological signals, for example by measuring voltages of acquired EEG signals.

Determining the topographic spectral power maps may involve applying a Fast Fourier transformation.

Herein, clustering may refer to any unsupervised method of determining reference states based on a topographic spectral power maps. After clustering, individual topographic spectral power maps (TSP maps), may be assigned to one of the determined clusters based on a similarity measure, preferably the same similarity measure that is used for determining the clusters. In the following, the TSP maps for all channels as an entirety is also referred to as TSP matrix.

Identifying the , among the plurality of macrostates, a default macrostate as the macrostate which shows a largest suppression during stimulation is preferably carried out by considering both the target stimulation and the reference stimulation.

The determined salient reactivity may be output as a by a numeric index, in the following also referred to as salient EEG reactivity (SER).

The target sensory stimuli could be applied in alternating sequence with reference sensory stimuli, not necessarily of the same sensory modality or complexity.

In embodiments of the method of the first aspect, the oscillatory electrical activity of the brain, measured by multi-channel EEG, can be decomposed into a sequence of N clusters of similar topographic spectral power distribution referred to as EEG macrostates at clustering level N. The method can identify the default EEG macrostate as the macrostate X (1<X<=N) which shows the largest suppression during sensory stimulation across clustering levels and stimuli, referred to as default EEG reactivity (DER) and in embodiments calculated as DER= PREₓ - STIM_{X}, where PRE_{X} and STIM_{X} are the occurrence probabilities before and during stimulation respectively (Nita et al. , 2016). Having measured DER to target stimuli (DER_{T}) and to reference stimuli (DER_{R}) using the same default EEG macrostate, SER can be calculated preferably as SER= DER_{T}- DER_{R}. The method of the first aspect can be applied to a broad range of fields interested in measuring sensory stimulation salience such as, but not limited to, neurology, psychiatry, neuroscience, and psychology. For validation purposes, an embodiment will be presented below comparing SER to auditory stimulation by the subject's own name (SON) in healthy volunteers and post-stroke comatose patients.

Specifically, embodiments of the method of the first aspect can quantify the contribution of stimulus salience to EEG reactivity, by a numeric index termed salient EEG reactivity (SER). The method of the first aspect can be applied to a broad range of fields interested in measuring sensory stimulation salience such as, but not limited to, neurology (e.g. disorders of consciousness), psychiatry (e.g. schizophrenia), neuroscience (e.g. brain networks), and psychology (e.g. attentional models).

The frequency of occurrence of the macrostate may be seen as a measure of the probability of this macrostate.

In an embodiment of the method of the first aspect, during a stimulation trial, the target sensory stimulus is applied during a stimulation period which comes after a first period without stimulation and before a second period without stimulation, wherein a duration of the first period and a duration of the second period are at least as long as a duration of the stimulation period.

In an embodiment of the method of the first aspect, the frequency bands include the delta, theta, alpha and beta frequency bands.

In an embodiment of the method of the first aspect, the method further comprises a step of determining the plurality of time points as time points that correspond to extremes of a variation within the topographic spectral power maps. For example, it can be determined at which time points the standard deviation of the topographic spectral power maps is highest and these time points can be used as the plurality of time points. This embodiment has the advantage that a computational effort for the processing of the data is reduced.

In an embodiment of the method of the first aspect, the clustering the topographic spectral power maps comprises performing hierarchical clustering using a cosine distance metric.

Experiments have shown that the cosine distance metric is particularly useful for clustering the high-dimensional EEG data.

In an embodiment of the method of the first aspect, the method is performed for a plurality of trials, and the clustering is performed across the plurality of trials. In other words, topographic spectral power maps, TSP, maps may be determined for time points of a plurality of trials and then clustering may be performed over the entire set of TSP maps.

In an embodiment of the method of the first aspect, the at least one target sensory stimulus comprises a plurality of stimuli, preferably of different sensory modalities. For example, the sensory modalities may include audio stimuli, visual stimuli, haptic stimuli and smell stimuli.

Preferably, the plurality of stimuli comprise target and reference stimuli.

Preferably, the plurality of stimuli of different sensory modalities is applied in an alternating manner.

In an embodiment of the method of the first aspect, the method further comprises optimizing an average of the salient reactivity across trials of a stimulus type, to determine an optimum number of classes for the clustering. For example, a clustering algorithm may require that the number of classes to be clustered is provided as input to the clustering algorithm. Different clustering levels (corresponding to different maximum numbers of clusters) may give results of different quality. Hence, this embodiment has the advantage that an optimum clustering level can be determined.

In an embodiment of the method of the first aspect, the method further comprises displaying the default macrostate as a sequence of Z-scaled topographic power maps, which is also referred to as the Default EEG macrostate spectral fingerprint. This embodiment has the advantage that a user can visually inspect the topographic power maps.

A second aspect of the present invention provides an apparatus for determining a salient reactivity of at least one target sensory stimulus applied to a subject, the apparatus comprising:
- an obtaining unit for obtaining physiological signals from the subject during a stimulation with the target sensory stimulus and during a stimulation with a reference sensory stimulus, the physiological signals comprising a plurality of channels, wherein the physiological signals are EEG signals,
- a determining unit for determining, at a plurality of time points of the physiological signals, topographic spectral power maps, wherein a topographic spectral power map comprises spectral powers of frequency bands of the plurality of channels,
- a clustering unit for clustering the topographic spectral power maps to obtain a plurality of macrostates,
- an identification unit for identifying, among the plurality of macrostates, a default macrostate as a macrostate which shows a largest suppression during stimulation, wherein a suppression of a macrostate during a sensory stimulus is determined as a difference between the probability of occurrence of the macrostate during the sensory stimulus compared to the probability of occurrence of the macrostate in a period before the sensory stimulus, and
- a difference unit for determining the salient reactivity based on a difference between the suppression of the default macrostate during the target sensory stimulus and a suppression of the default macrostate during the reference sensory stimulus.

The apparatus of the second aspect can be configured to carry out the method of the first aspect or of one of the embodiments of the first aspect.

A further aspect of the present invention provides a computer-readable storage medium storing program code, the program code comprising instructions that when executed by a processor carry out the method of the first aspect or of one of the embodiments of the method of the first aspect.

### BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the disclosure. The scope of protection is defined by the appended claims.
- FIG. 1: shows a block diagram of an apparatus used for SER measurements,
- FIG. 2: shows a flow chart of a method for SER calculation,
- FIG. 3: illustrates an example multichannel EEG recording (A) of a STIM_{SON} target stimulation trial (B),
- FIG. 4: shows an example EEG channel from the trial of FIG. 3 in more detail detailed (A) to illustrate its power spectral density spectrogram (B) and the subsequent grouping in frequency bands (C),
- FIG. 5: illustrates an example representing the topographic spectral power (TSP) matrix of the trial of FIG. 3 combined across all channels (A) and its corresponding topographic standard deviation (B),
- FIG. 6: illustrates an example decomposition of the FIG.3 trial into an optimal number of macrostates to maximize DER (A), the binary time-course of each identified macrostate during the trial (B) and the macrostate strip representation (C),
- FIG. 7: is an example summary of the DER analysis for STIM_{SON} versus STIM_{rSON} is presented as the occurrence of default EEG class in each trial (A), the DER of each trial(B), and the default EEG macrostate spectral fingerprint (C),
- FIG. 8: shows the average DER measurements (A) and the default EEG macrostate spectral fingerprints (B) for the Study 1,
- FIG. 9: illustrates the SER measurements for Study 1 and Study 2 presented separately (top) and pooled (bottom), and
- FIG.10: illustrates a receiver operating characteristic (ROC) analysis comparing SER with DER measures.

### DETAILED DESCRIPTION OF EMBODIMENTS

The foregoing descriptions are only implementation manners of the present invention, the scope of the present invention is not limited to this. Any variations or replacements can be easily made through person skilled in the art. Therefore, the protection scope of the present invention should be subject to the protection scope of the attached claims.

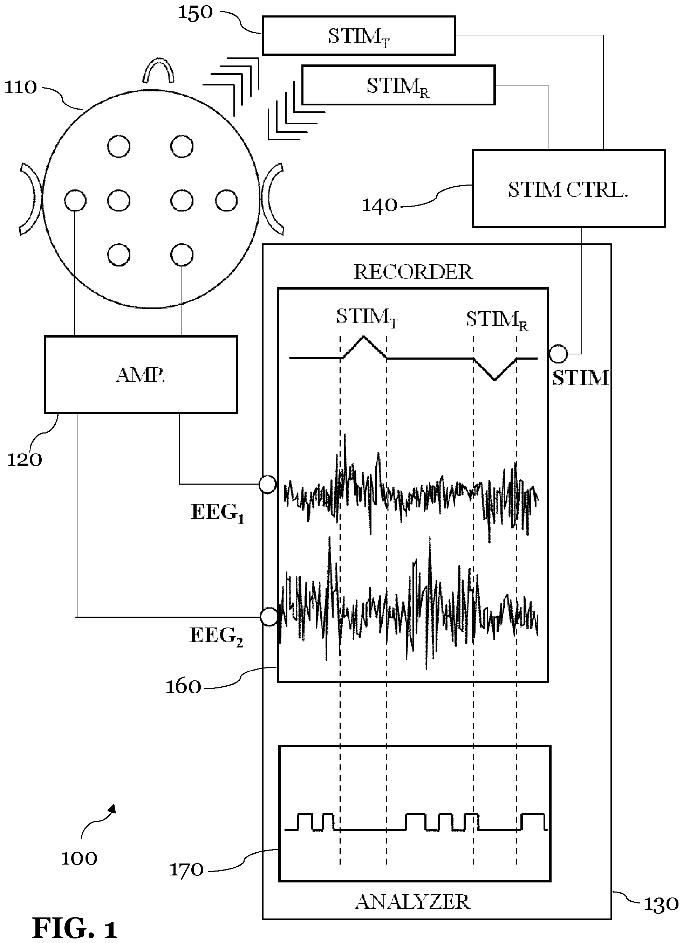

FIG. 1 is a block diagram of an apparatus 100 used for SER measurements. The apparatus comprises a multi-channel EEG electrode recording montage 110. For illustration purposes only two channels are indicated, referred to as EEG₁ and EEG2. The recorded signals are amplified and digitized in an amplifier 120 for a central processing unit 130 which can control programmatically, via a stimulus control unit 140, a multimodal stimulator 150 that can deliver alternatingly target stimuli (STIM_{T}) and reference stimuli (STIM_{R}). The central processing unit 130 (e.g. a personal computer) can have software for recording/displaying/archiving 160 multichannel EEG in parallel with a synchronized "STIM" channel where the timing and type of stimuli used can be identified. Preferably, an additional analysis software 170 is used to compute SER_{T-R} of the STIM_{T} as compared to STIM_{R}.

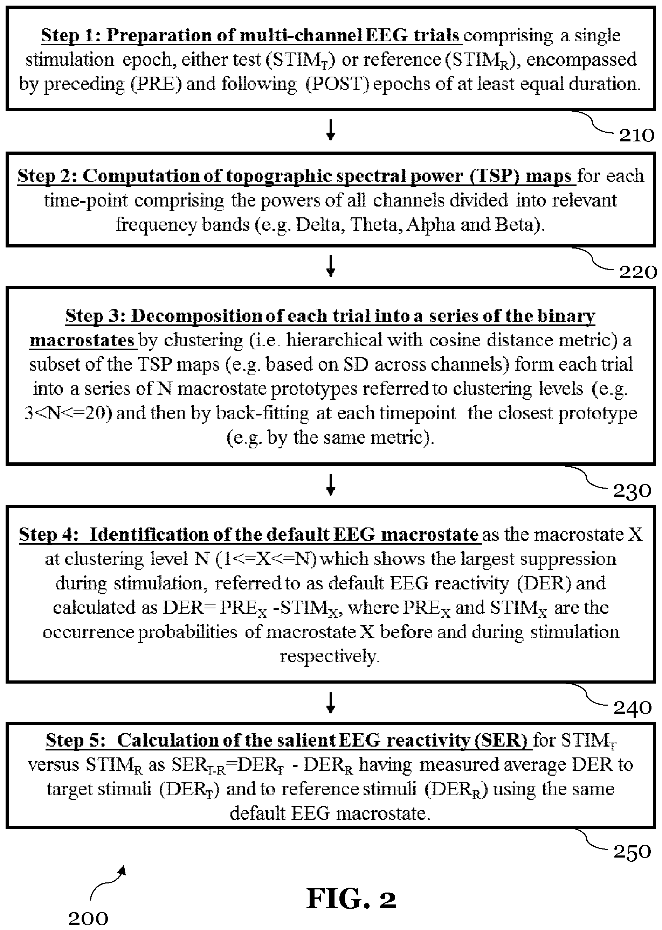

FIG. 2 shows a flow chart of a method 200 for SER calculation. The method comprises a first step 210 of preparing multi-channel EEG trials comprising a single stimulation epoch. The method comprises a second step 220 of computing the topographic spectral power (TSP) maps for each trial., In a third step 230, each trial is decomposed into a series of the binary macrostates. In a fourth step 240 the default EEG macrostate is identified, wherein the default EEG macrostate may be shared across trials. Finally, in a fifth step 250 calculation of the salient EEG reactivity (SER) is performed.

In detail, in a preferred embodiment, the method comprises the following five consecutive steps:
***Step 1**: **Preparation of multi-channel EEG trials*** comprising a single stimulation epoch, either target (STIM_{T}) or reference (STIM_{R}), encompassed by preceding (PRE) and following (POST) epochs of at least equal duration.
***Step 2: Computation of the topographic spectral power (TSP) maps*** for each time-point comprising the powers of all channels divided into relevant frequency bands (e.g. Delta, Theta, Alpha and Beta).
***Step 3: Decomposition of each trial into* α *series of the binary macrostates*** by clustering (for example hierarchical with cosine distance metric) a subset of the TSP maps (e.g. based on standard deviation across channels) from each trial into a series of N macrostate prototypes referred to clustering levels (e.g. 3<N<=20) and then by back-fitting at each timepoint the closest prototype (e.g. by the same metric).
***Step 4: Identification of the default EEG macrostate*** as the macrostate X at clustering level N (1<=X<=N) which shows the largest suppression during stimulation, referred to as default EEG reactivity (DER) and calculated as DER= PRE_{X} -STIM_{X}, where PRE_{X} and STIM_{X} are the occurrence probabilities of macrostate X before and during stimulation respectively.
***Step 5: Calculation of the salient EEG reactivity (SER)*** for STIM_{T} versus STIM_{R} as SER_{T-R}=DER_{T} - DER_{R} having measured average DER to target stimuli (DER_{T}) and to reference stimuli (DER_{R}) using the same default EEG macrostate.

In an attempt to reconcile the frequency fingerprint with the voltage-domain microstate decomposition theories the EEG decomposition is introduced in power-domain states with distinct topographic power distributions, herein also referred to as "macrostates", as a way to identify large-scale electrophysiological correlates of mental states. Thus, the EEG activity can be described as a sequence of "frames" where task-related macrostates alternate with resting macrostates, in keeping with the experimental findings that both perception and RSN activity occur fragmented in time.

Although the default mode network, DMN, the salience network, SN, and the executive-control network, ECN, show independent activities at rest, their activities coordinate during stimulation, such as the activation of the SN by a sensory stimulus would, in turn, activate the ECN and deactivate the DMN. This coupling can be stronger in the context of salient stimuli. Thus, rather than attempting to identify the EEG macro-state related to the SN activation, embodiments of the method of the first aspect measures salience by measuring deactivation of the EEG macrostate related to DMN.

The activity of each macrostate can be described as a binary signal, where the occurrence probability in a certain time epoch can be measured similarly to the suppression ratio of the alternating burst-suppression EEG patterns observed in deep comatose states. Thus, a macrostate reactivity to sensory stimulation could be described similarly to a measure of burst-suppression reactivity as the decrease in occurrence probability during sensory stimulation as compared to the occurrence probability prior to stimulation. Considering that DMN is largely task-negative (e.g. suppressed by sensory activity), the macrostate which shows the largest decrease in occurrence probability is referred to as default EEG macrostate, and consequently, its reactivity is referred to as default EEG reactivity (DER).

Since salience is a relative term - the salience of a target stimulus (STIM_{T}) should therefore be considered relative to (or in the context of) a reference stimulus (STIM_{R}). Thus, we refer to the salience SER_{T-R} for STIM_{T} versus STIM_{R}. The general principle is the decomposition of multichannel EEG into into a series of N alternating clusters of similar topographic power spectral distribution also referred to as EEG macrostates. Then, the macrostate showing the largest suppression during stimulation for both STIM_{T} and STIM_{R}, referred to as the default EEG macrostate is identified. The magnitude of the decrease in occurrence probability during stimulation (measured in percent), referred to as default EEG reactivity (DER), is used to calculate SER, as SER_{T-R}=DER_{T} - DER_{R}. Numerically, SER has a maximum of 100%, which is theoretically attained when the EEG before the stimulation is composed 100% of time by the default EEG macrostate, which is then completely abolished during STIM_{T} (0%) whereas its occurrence remains unaffected (remains 100%) during STIM_{R}. To avoid imposing a hypothesis bias, no methodological assumptions are constrained so that the STIM_{T} would be more salient than STIM_{R}. Therefore, only a positive SER_{T-R} can concur such that STIM_{T} is more salient than STIM_{R} and SER_{T-R} = - SER_{R-T}.

To measure SER, synchronized EEG responses are delivered and compared to a sequence of alternating sensory stimuli, not necessarily of the same modality or complexity. A suitable apparatus for SER recording could readily be assembled by combining currently available technologies (FIG. 1), ensuring the wide availability and reduced implementation costs of the proposed invention. Clinical EEG recording, and sensory stimulation should be carried out in agreement with current safety and methodological practices. The resulting recording from each subject should be presented as a multichannel EEG recording with a synchronized "STIM" channel where the timing and type of stimuli used could be accurately identified.

Prior art methods are primarily data-driven so that a combination of quantitative EEG measures are optimized to discriminate a particular reactive brain behavior. In contrast, embodiments of the presented method provide an algorithm-driven approach to measure EEG reactivity, referred to as DER. Furthermore, prior art EEG reactivity measures are either sensory-modality specific or they rely on direct brain stimulation which does not discriminate between stimuli and does not allow for salience measurements. In contrast, embodiments focus on suppression of a resting EEG feature, rather than activation by a specific sensory modality, thus enabling salience measurements of stimuli of different sensory modality or complexity.

Previously proposed DER indices were measured independently for each stimulation trial and did not provide a shared default EEG reactivity macrostate among trials that is required to calculate SER. Also, the present invention focuses the TSP clustering on broad frequency bands, rather than considering all independent spectral components which has the advantage of increasing the physiological significance whereas reducing the computational load on multi-channel EEG. Finally, embodiments of the presented method provide an automatic method to select the optimal number of clustering levels by maximizing DER among trials rather than assuming an empirical decomposition level, which also makes it more robust to recording artefacts.

Step 3 can involve topographic clustering of multi-channel EEG relates to microstate EEG analysis. Nevertheless, microstates are clustered in the voltage-domain which are not correlated with measurements in the power-domain. Furthermore, the microstates are identified based on their topographic features and the ability to explain EEG variance, whereas the default EEG macrostate is defined based on its behavior during sensory stimulation.

### Embodiment background

The salience network is particularly linked to consciousness especially in the context of auditory stimuli. Accumulating EEG evidence suggest that one of the most salient stimuli across different states of consciousness is the subjects own name (SON), responses to which seem to fade latest during sleep. Apart for assessment of the level of consciousness, EEG changes to SON were also found to have prognostic value in brain injury, although the single subject variability was large. Studies with SON stimuli were typically carried out using novelty event-related potentials and the subsequent analysis of post-stimulus time-frequency analysis. Nevertheless, such task-dependent alterations found in background EEG oscillations, classically referred to as event-related synchronization and desynchronization, were investigated over short time scales (up to a couple of seconds) and could not distinguish salience from stimulus awareness.

A validation of embodiments of the presented method involves measuring SER of a SON target stimulus versus a reference stimulus comprised of a meaningless noise of the same energy obtained by reversing SON (rSON), in healthy volunteers and comatose patients. As a coma model we selected the post-stroke comatose patients, because anaesthetic coma can have confounding effects on salience processing reactivity. Primarily, it is tested whether the SER_{SON-rSON} was positive (identifying SON as the salient stimulus) and whether the SER_{SON- rSON} is reduced in coma.

### Experimental validation

Neurophysiological recordings were obtained from 15 healthy control volunteers and 15 deeply comatose post-stroke patients (mostly acute ischemic middle cerebral artery stroke) by the Glasgow Coma Scale and with different hemispheric injury localizations. Most included patients deceased within 7 days after their stroke so that establishing the prognostic value of SER was beyond the scope of this cohort.

The studies were carried out during 2016-2018 at the Neurology Department, University Emergency Hospital, Bucharest, Romania, with approval of the institutional ethics committee as well as informed consent, in agreement to the World Medical Association (WMA) declaration of Helsinki on ethical principles for medical research involving human subjects (last amended at the 64th WMA General Assembly, Fortaleza, Brazil, October 2013).

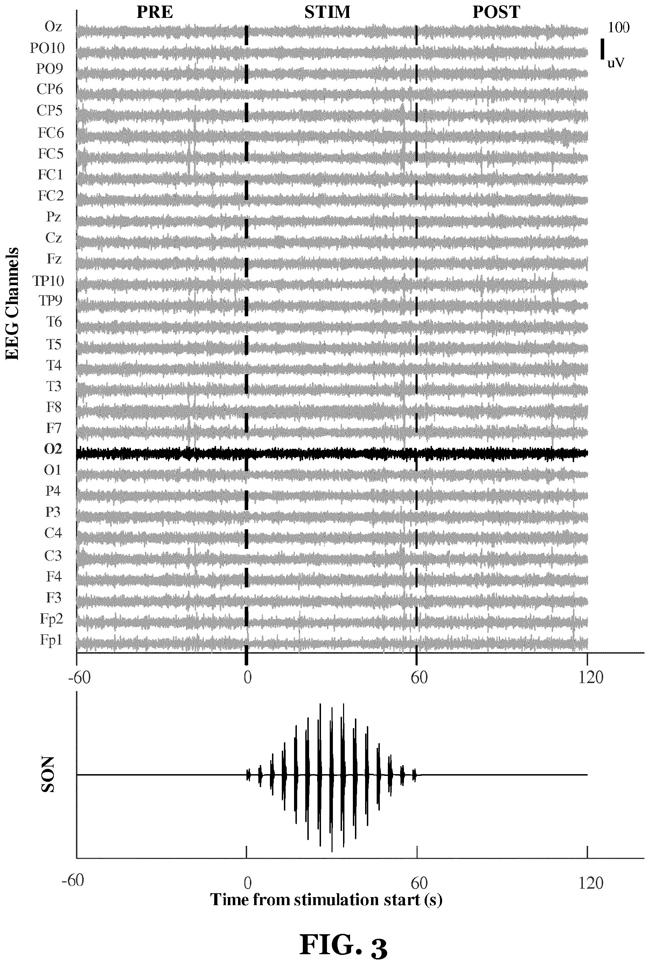

Auditory target SON stimuli were generated using a commercial Romanian language text-to-speech synthesizer to avoid emotional salience confounders to familiar voices. The stimuli (FIG. 3B) were repeated over a 1-minute stimulation epoch (STIM_{SON}) to minimize novelty effect, consistent with our previous studies of coma reactivity. Furthermore, the stimulation intensity was windowed over STIM_{SON} (FIG. 3B) to minimize the confounding effects of auditory startle. The refence 1-minute stimulation epoch was obtained by inversing the target SON epoch (STIMr_{SON}). The stimuli were delivered over a pair of bone conduction headphones. The STIM_{SON} epochs were presented in alternation with STIMr_{SON} epochs with at least 2-minute intervals, to ensure the ability to segment each trial to include PRE and POST epochs of at least 1-minute (FIG. 2, step 1).

As a rule, we aimed to record 3 SON and 3 rSON trials amounting to a standard 20-minute EEG recording. Each subject was investigated once, except one volunteer that was recoded twice. In one patient, only SON stimulation was recorded so that no SER measurement could be carried out.

Electroencephalographic (EEG) recordings were obtained from scalp electrodes comprised in a suitable 32 channels "EASY-CAP" (EASYCAP GmbH, DE) in agreement with the international standards for EEG electrode placement. The electrode contact impedance was decreased using "Signa gel" (SignaGel Electrode Gel: Parker Laboratories, Inc, USA) electrode-or "Ten20" conductive paste (Weaver and Company Inc, USA).

Both volunteers and patients were randomly assigned in 2 study groups. In Study 1, the EEG was recorded with a commercial EEG machine, namely NicoletOne ™ EEG System - Neuro (Natus Inc, USA). The auditory stimulus generation and synchronization (FIG.1) in this case was carried out with an external PC running MATLAB (Mathworks Inc USA) via the Psychophysics Toolbox (Brainard, 1997), which allows accurate timing of auditory signals. In Study 2, the EEG was recorded with a custom-made system referred to as Neurophysiological EEG Reactivity Monitor (NERMO) integrating both stimulation and recording in a single hardware-software solution based on the commercial 24-bit analog-to-digital converter for biopotential measurements ADS1299 chip (Texas Instruments, USA) designed in agreement with EEG recording performance standards IEC-60601-2-26 and IEC 60601-2-27. To further minimize novelty and expectancy effect, stimulation trials were alternated in order rSON/SON for Study 1 and SON/rSON for the Study 2.

The EEG analysis required for SER measurements was carried out offline using a custom plugin, referred to as NERMO, for the open source EEGLAB toolbox environment (Delorme et al. , 2004). The group statistical parameters and tests are presented when used.

### Example analysis

A typical analysis for a volunteer in Study 1 is detailed in FIGs 3 to 7. FIG. 3 presents a multichannel EEG recording (FIG. 3A) of the SON stimulation trial (FIG. 3B) with largest DER comprised of PRE, STIM and POST epochs of 1-minute, prepared after Step 1 in FIG.2. The EEG is presented in unipolar montage using the ear as reference, at 256 Hz sampling rate and filtered in the 1-30Hz band.

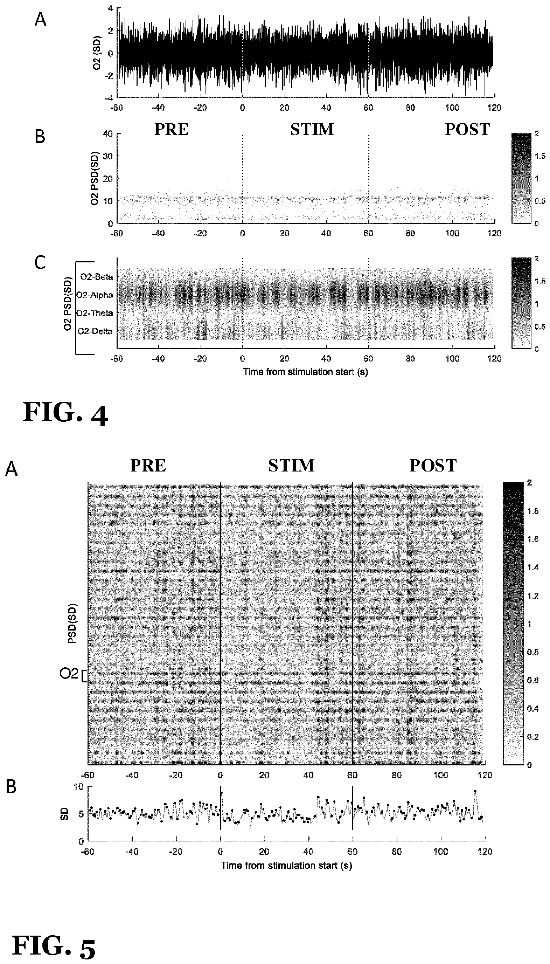

In FIG. 4 is detailed the power analysis for the right occipital channel 02 in FIG. 3. The EEG in FIG. 4A is presented normalized to its standard deviation (SD) across the trial, which ensures equal weight to all the channels/trials. FIG.4B presents the time-frequency power spectral density spectrogram (measured over a sliding 2-second window with no overlap) of the recording in FIG.4A. Note the very robust Alpha band activity appearing as a horizontal line. The corresponding recording cumulated in 4 power bands for Delta, Theta, Alpha and Beta activities is presented in Fig. 4B. These powers are further cumulated for all channels in a topographic spectral power (TSP) matrix (FIG. 5A). Thus, each column of the TSP matrix represents a TSP map and is comprised of NC x NB rows, where NC is the number of channels and NB is the number of bands.

To reduce the computational load, not all TSP maps are included in clusterization. In microstate analysis it was proposed that the selection should be carried out at dissimilarity maxima e.g. based on the Global Field Power (GFP) signal. Here the same approach was used, however, considering the extremes of the topographic variation within TSPs, measured by SD. These reference TSP maps were marked with dots in FIG. 5B.

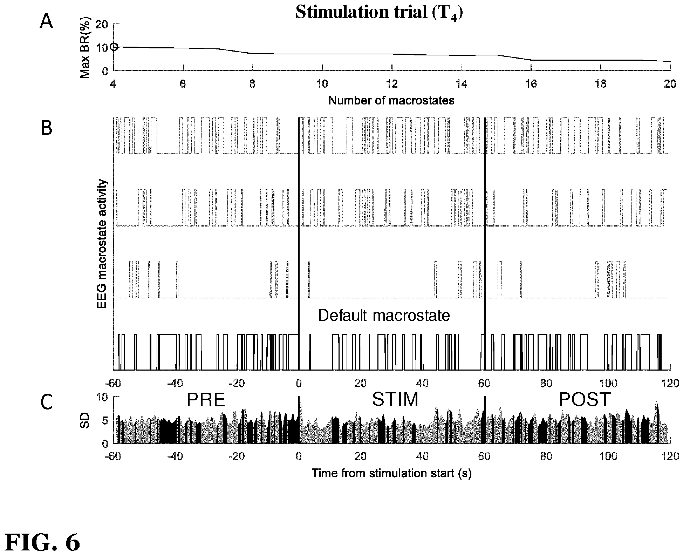

In FIG. 6 is presented the resulting clustering of the combined reference TSP matrix across trials (FIG. 2, step 3) by hierarchical clustering which is independent on randomization as the measures used for microstate clustering (Pascual-Marqui et al. , 1995). Class prototypes (macrostates) were then identified as the TSP maps having the largest similarity to all the class members. These shared TSP map prototypes were then back-fitted to each time-sample of the trials. Hierarchical clusterization and back-fitting of TSP maps were carried out for N=4 to 20 levels (maximum number of clusters) using the same similarity distance measure in NC x NB dimensions. Given the high dimensionality of the data a cosine distance metric was chosen.

The time-course of a macrostate X is assimilated to a binary signal, where its logical 1 (depicted upwards) indicates the occurrence of that macrostate. The binary reactivity (BR) of the macrostate X can then be calculated as BR_{X} = PRE_{X} - STIM_{X}, where PRE_{X} and STIM_{X} are the occurrence probabilities of macrostate X (fraction of time spent in state X) before and during stimulation respectively, by analogy with measures introduced for binary burst-suppression EEG reactivity. The BRs are then averaged across trials and clustering levels for both SON and rSON stimuli. The maximum average BR for each clustering level (DER) is presented in FIG. 6A. The overall maximum DER, in this case occurring at clustering level N=4, is indicated with a circle. It should be emphasized that the method does not enforce that this maximum should occur for SON stimuli.

Having identified the optimal clustering level, the corresponding time-course of each of the four macrostates is presented in FIG. 6B, ordered by BR from top (smallest) to bottom (largest). The occurrence of the default EEG macrostate (with the largest DER) is depicted in black. In FIG. 6C, for analogy with the microstate analysis presentation, the macrostates are presented in time sequence based on the topographic SD of the trial (FIG. 5B) referred to as the "macrostate strip representation", using the same color code as in FIG 6B.

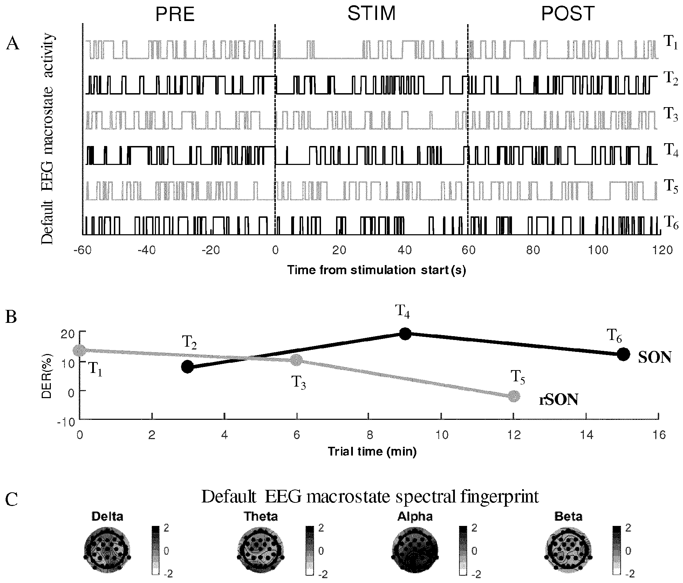

In FIG. 7 the DER analysis is summarized for all the 6 trials (T₁,..., T₆) recorded for the subject. The time-course of the binary class corresponding to the default EEG macrostate is presented in FIG. 7A. The corresponding DER for each SON and rSON trial is presented in FIG. 7B. These values are then averaged across trials of similar stimulus to measure DER_{SON} and DER_{rSON} measures which are used to calculate DER_{SON-rSON}= DER_{SON} - DER_{rSON}.

To illustrate the default EEG macrostate that is shared across trials, its topographic distribution maps is presented for corresponding Delta, Theta, Alpha and Beta bands (FIG. 7C) using the standard topoplot() function in the EEGLAB toolbox. The values at each location (V) were scaled across the maps as Z-values = (V-mean)/SD, referred to hereafter as the "default EEG macrostate spectral fingerprint".

### Validation results

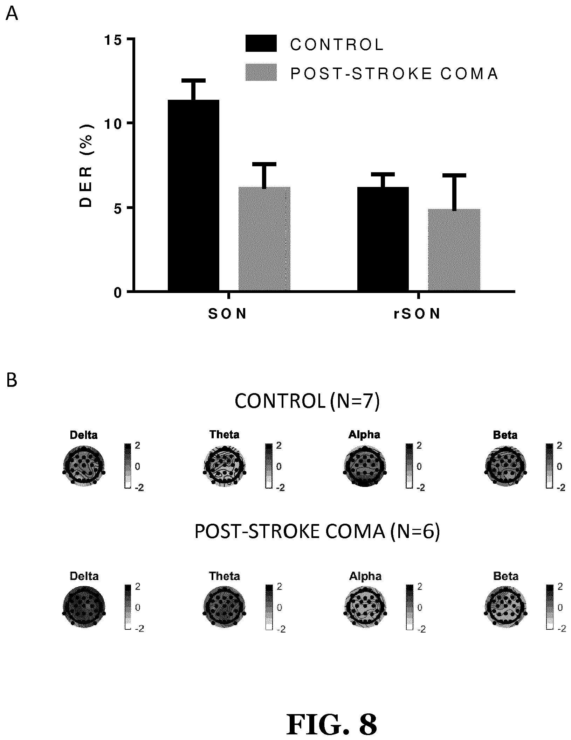

The average DER measurements for the Study 1 are presented in FIG. 8A. Error bars represent SEM. In controls, the DER_{SON} was 11.3 ± 1.3 % which was nearly double than the DER_{rSON} of 6.2 ± 0.8 % (P=0.016, Wilcoxon matched-pairs signed rank test). In comatose patients, DER_{SON} dropped to 6.1 ± 1.4 % (P=0.022, Mann Whitney test). Taken together, these data supported the original hypothesis on SON salience. Furthermore, in the comatose patients, DER_{SON} became undistinguishable not only from DERr_{SON} but also from DER_{rSON} in controls (Ordinary Two-way ANOVA with stimulus type and subject group as factors, P>0.₉ Bonferroni's multiple comparisons test). This "plateau" reflected most likely the contribution to measured DER of the primary activation of the sound areas, which are independent of conscious sound perception, and, as such, not influenced by salience.

The default EEG macrostate spectral fingerprints from which DER was calculated for Study 1 (FIG. 8A) appeared, markedly different between volunteers and comatose patients (FIG. 8B). In volunteers, there was a dominant posterior Alpha and Central beta activity consistent to previous reports. In contrast, in patients the activity of the detected default EEG macrostate was mainly confined to the Delta and Theta, consistent with the ischemic EEG slowing detected in middle cerebral artery stroke patients as well as experimental studies. These data raise the hypothesis that the EEG fingerprint of the DMN can vary according to the brain condition, which could account for the variability reported in previous studies. If so, it becomes relevant to distinguish DMN not by its signature in controls but rather by its task-negative behaviour, as is done by embodiments of the present invention.

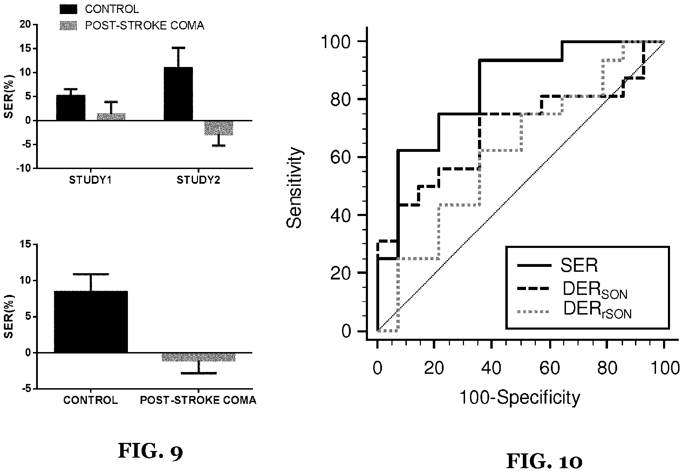

The SER measurements for Study 1 were similar to SER measurements in Study 2 which were carried out in different subjects, using a different hardware, and a different SON/rSON alternance (FIG. 9A). In the pooled analysis SER_{SON-rSON} was 8.5 ± 2. 5 % in controls and was abolished in comatose patients -1 ± 1.8 % (P=0.0011, Mann Whitney test) consistent with previous observations of deactivation of the DMN as a marker of impaired consciousness. It should be noted that the number of macrostates from which SER was calculated was also similar between stimulus type and subject group (data not shown), with a pooled value of 8.8 ± 0.8 macrostates.

To compare the discriminative ability of SER_{SON-rSON}, a receiver operating characteristic (ROC) analysis was carried out. It was found that SER_{SON-rSON} ROC curve at maximal Youden index J of 0.58 indicated a very high discriminative sensitivity of 93.75 and a good specificity of 64.29. The relatively lower specificity was consistent with the theory that there are different levels of conscious awareness. Furthermore, it was found that SER_{SON-rSON} had an Area under the ROC curve (AUC) of 0.84 which was higher (FIG. 10) than either DER_{SON} and DERr_{SON} (P<0.05). This indicated that SER measurements are more discriminative than DER measurements, possibly by reducing individual variation factors.

Embodiments of the present invention provide a theoretically based electroencephalographic (EEG) method and apparatus for measuring sensory stimulation salience of a target sensory stimulus (STIM_{T}) versus a reference sensory stimulus (STIM_{R}) comprising:
- An apparatus for delivering STIM_{T} and STIM_{R} while recording EEG.
- A method for identifying a default EEG macrostate.
- A method to measure default EEG reactivity (DER) for STIM_{T} and STIM_{R}.
- A method to calculate a numerical index measuring the salience of STIM _{T} versus STIM_{R} based on DER, referred to as the salient EEG reactivity (SER).

Preferably, the apparatus can deliver alternating stimuli of different sensory modalities and complexity synchronized to multi-channel EEG recording. Preferably, the EEG can be decomposed in a series of classes with similar topographic power distribution referred to as macrostates. Preferably, the macrostate showing the largest occurrence suppression during sensory stimulation is referred to as the Default EEG macrostate. Preferably, the default EEG reactivity (DER) for STIM_{T} (DER_{T}) and STIM_{R} (DER_{R}) can be calculated in the following steps:
- Preparation of multi-channel EEG trials comprising a single stimulation epoch (either STIM_{T} or STIM_{R})
- Computation of the topographic spectral power matrix (TSP) for each trial
- Decomposition of each trial into a series of the binary macrostates
- Identification of the default EEG macrostate common to STIM_{T} and STIM_{R}
- Measurement of DER_{T} and DER_{R}

Preferably, the trials the STIM epoch is encompassed by preceding (PRE) and following (POST) epoch of at least equal duration. Preferably, the TSP is calculated by combining the broad band spectrograms for each EEG channel. Preferably, reference TSPs for each channel are identified according to topographic variation. Further preferably, the reference TSPs from each trial are clustered across trials to determine macrostate prototypes. In further embodiments, the macrostate prototypes are back-fitted to the trials to derive time-course of occurrence of each macrostate depicted as binary signals.

In further embodiments, the default EEG macrostate is identified as the macrostate with the largest decrease in occurrence probability from PRE to STIM epochs, referred to as DER. The average DER across all the trials of a stimulus type (DER_{T} or DER_{R}) can be maximized across a series of clustering levels (maximum number of classes).

The default EEG macrostate can be displayed as a sequence of Z-scaled topographic power maps referred to as "default EEG macrostate spectral fingerprint". The salience index can be calculated as SER_{T-R}=DER_{T} - DER_{R}, measured in percent.

### REFERENCES

Brainard DH. The Psychophysics Toolbox. Spat Vis. 1997;10:433-6.
Britz J, Van De Ville D, Michel CM. BOLD correlates of EEG topography reveal rapid resting-state network dynamics. Neurolmage. 2010;52:1162-70.
Delorme A, Makeig S. EEGLAB: an open source toolbox for analysis of single-trial EEG dynamics including independent component analysis. J Neurosci Methods. 2004;134:9-21.
Ilie A, loan S, Zagrean L, Moldovan M. Better to be red than blue in virtual competition. Cyberpsychol Behav. 2008;11:375-7.
loan S, Sandulache M, Avramescu S, Ilie A, Neacsu A, Zagrean L, et al. Red is a distractor for men in competition. Evolution and Human Behavior. 2007;28:285-93.
Mantini D, Perrucci MG, Del Gratta C, Romani GL, Corbetta M. Electrophysiological signatures of resting state networks in the human brain. Proc Natl Acad Sci U S A. 2007;104:13170-5.
Nita D, Moldovan M, Sharma R, Avramescu S, Otsubo H, Hahn C. The burst-suppression EEG pattern in comatose children is reactive to photic stimulation. European Journal of Neurology. 2016;23:273.
Pascual-Marqui RD, Michel CM, Lehmann D. Segmentation of brain electrical activity into microstates: model estimation and validation. IEEE Trans Biomed Eng. 1995;42:658-65.
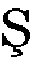 erban C-A, Barboric 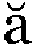 A, Roceanu A-M, Mindru 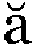 I-R, Ciurea J, Z 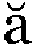 grean A-M, et al. EEG Assessment of Consciousness Rebooting from Coma. In: Opris I, Casanova M, F., editors. The Physics of the Mind and Brain Disorders. Switzerland: Springer; 2017. p. 361-81.

## Claims

1. A computer-implemented method (200) for determining a salient reactivity of at least one target sensory stimulus applied to a subject, the method comprising:
- obtaining (210) physiological signals from the subject during a stimulation with the target sensory stimulus and during a stimulation with a reference sensory stimulus, the physiological signals comprising a plurality of channels, wherein the physiological signals are electroencephalographic (EEG) signals,
- determining (220), at a plurality of time points of the physiological signals, topographic spectral power maps, wherein a topographic spectral power map comprises spectral powers of frequency bands of the plurality of channels,
- clustering (230) the topographic spectral power maps to obtain a plurality of macrostates,
- identifying (240), among the plurality of macrostates, a default macrostate as the macrostate which shows a largest suppression during stimulation, wherein a suppression of a macrostate during a sensory stimulus is determined as a difference between the probability of occurrence of the macrostate during the sensory stimulus compared to the probability of occurrence of the macrostate in a period before the sensory stimulus, and
- determining (250) the salient reactivity based on a difference between the suppression of the default macrostate during the target sensory stimulus and a suppression of the default macrostate during the reference sensory stimulus.

2. The method (200) of claim 1, wherein during a stimulation trial, the target sensory stimulus is applied during a stimulation period which comes after a first period without stimulation and before a second period without stimulation, wherein a duration of the first period and a duration of the second period are at least as long as a duration of the stimulation period.

3. The method (200) of one of the previous claims, wherein the frequency bands include the delta, theta, alpha and beta conventional EEG frequency bands.

4. The method (200) of one of the previous claims, further comprising a step of determining the plurality of time points as time points that correspond to extremes of a variation within the topographic spectral power maps.

5. The method (200) of one of the previous claims, wherein the clustering (230) the topographic spectral power maps comprises performing hierarchical clustering using a cosine distance metric.

6. The method (200) of one of the previous claims, wherein the method is performed for a plurality of trials, and the clustering is performed across the plurality of trials.

7. The method (200) of one of the previous claims, wherein the at least one target sensory stimulus comprises a plurality of stimuli.

8. The method (200) of claim 7, wherein the plurality of stimuli is applied in an alternating manner.

9. The method (200) of one of the previous claims, further comprising optimizing an average of the salient reactivity across trials of a stimulus type, to determine an optimum number of macrostate classes.

10. The method (200) of one of the previous claims, further comprising displaying the default macrostate as a sequence of Z-scaled topographic power maps.

11. An apparatus (100) for determining a salient reactivity of at least one target sensory stimulus applied to a subject, the apparatus comprising:
- an obtaining unit (110) for obtaining physiological signals from the subject during a stimulation with the target sensory stimulus and during a stimulation with a reference sensory stimulus, the physiological signals comprising a plurality of channels, wherein the physiological signals are EEG signals,
- a determining unit (170) for determining, at a plurality of time points of the physiological signals, topographic spectral power maps, wherein a topographic spectral power map comprises spectral powers of frequency bands of the plurality of channels,
- a clustering unit (170) for clustering the topographic spectral power maps to obtain a plurality of macrostates,
- an identification unit (170) for identifying, among the plurality of macrostates, a default macrostate as a macrostate which shows a largest suppression during stimulation, wherein a suppression of a macrostate during a sensory stimulus is determined as a difference between the probability of occurrence of the macrostate during the sensory stimulus compared to the probability of occurrence of the macrostate in a period before the sensory stimulus, and
- a difference unit for determining the salient reactivity based on a difference between the suppression of the default macrostate during the target sensory stimulus and a suppression of the default macrostate during the reference sensory stimulus.

12. A computer-readable storage medium storing program code, the program code comprising instructions that when executed by an apparatus as defined in claim carry out the method (200) of one of claims 1 to 10.

## Patentansprüche

1. Ein computerimplementiertes Verfahren (200) für die Bestimmung einer auffälligen Reaktivität von mindestens einem sensorischen Zielstimulus, der auf ein Objekt angewendet wird. Das Verfahren besteht aus:
- Empfang (210) physiologischer Signale vom Objekt während einer Stimulation mit dem sensorischen Zielstimulus und während einer Stimulation mit einem sensorischen Bezugsstimulus, wobei es sich bei den physiologischen Signalen um eine Vielzahl von elektroenzephalografischen (EEG) Signalen handelt,
- Bestimmung (220) von topografischen Spektralleistungsbildern zu verschiedenen Zeitpunkten der physiologischen Signale, wobei ein topografisches Spektralleistungsbild aus den spektralen Leistungen der Frequenzbänder einer Vielzahl von Kanälen besteht.
- Gruppierung (230) der topografischen Spektralleistungsbilder für die Bereitstellung einer Vielzahl von Makrozuständen,
- Erkennung (240) eines Standardmakrozustands unter der Vielzahl von Makrozuständen als Makrozustand, der während der Stimulation die größte Suppression zeigt, wobei eine Suppression eines Makrozustand während eines sensorischen Stimulus als Differenz zwischen der Wahrscheinlichkeit des Auftretens des Makrozustands während des sensorischen Stimulus in einem Zeitraum vor dem sensorischen Stimulus bestimmt wird, und
- Bestimmung (250) der auffälligen Reaktivität auf der Basis einer Differenz zwischen der Suppression des Standardmakrozustands während des sensorischen Zielstimulus und einer Suppression des Standardmakrozustands während des sensorischen Bezugsstimulus.

2. Das Verfahren (200) nach Anspruch 1, bei dem der sensorische Zielstimulus während eines Stimulationsversuchs in einem Stimulationszeitraum angewendet wird, der nach einem ersten Zeitraum ohne Stimulation und vor einem zweiten Zeitraum ohne Stimulation kommt, wobei die Dauer des ersten Zeitraums und die Dauer des zweiten Zeitraums mindestens so lang wie die Dauer des Stimulationszeitraums ist.

3. Das Verfahren (200) nach einem der vorherigen Ansprüche, wobei die Frequenzbänder die herkömmlichen Delta-, Theta-, Alpha- und Beta-EEG-Frequenzbänder umfassen.

4. Das Verfahren (200) nach einem der vorherigen Ansprüche, das einen Schritt zur Bestimmung der Vielzahl von Zeitpunkten als Zeitpunkte enthält, die den Extremen einer Variation innerhalb der topografischen Spektralleistungsbilder entsprechen.

5. Das Verfahren (200) nach einem der vorherigen Ansprüche, wobei die Gruppierung (230) der topografischen Spektralleistungsbilder die hierarchische Gruppierung mithilfe einer Kosinusabstandsmetrik einschließt.

6. Das Verfahren (200) nach einem der vorherigen Ansprüche, wobei das Verfahren bei einer Vielzahl von Versuchen und die Gruppierung über eine Vielzahl von Versuchen durchgeführt wird.

7. Das Verfahren (200) nach einem der vorherigen Ansprüche, wobei mindestens ein sensorischer Zielstimulus aus einer Vielzahl von Stimuli besteht.

8. Das Verfahren (200) nach Anspruch 7, wobei die Vielzahl der Stimuli abwechselnd angewendet wird.

9. Das Verfahren (200) nach einem der vorherigen Ansprüche, das außerdem die Optimierung eines Mittelwerts der auffälligen Reaktivität über mehrere Versuche mit einer bestimmten Stimulusart umfasst, um eine optimale Anzahl von Makrozustandsklassen zu bestimmen.

10. Das Verfahren (200) nach einem der vorhergehenden Ansprüche, das außerdem das Anzeigen des Standardmakrozustands als eine Sequenz der Z-skalierten topografischen Leistungsbilder umfasst.

11. Ein Gerät für die Bestimmung einer auffälligen Reaktivität von mindestens einem sensorischen Zielstimulus, der auf ein Objekt angewendet wird. Das Gerät besteht aus:
- einer Empfangseinheit (110) für den Empfang physiologischer Signale vom Objekt während einer Stimulation mit dem sensorischen Zielstimulus und während einer Stimulation mit einem sensorischen Bezugsstimulus, wobei es sich bei den physiologischen Signalen um eine Vielzahl von EEG-Signalen handelt,
- einer Bestimmungseinheit (170) zur Bestimmung von topografischen Spektralleistungsbildern zu verschiedenen Zeitpunkten der physiologischen Signale, wobei ein topografisches Spektralleistungsbild aus den spektralen Leistungen der Frequenzbänder einer Vielzahl von Kanälen besteht.
- einer Gruppierungseinheit (170) zur Gruppierung der topografischen Spektralleistungsbilder für die Bereitstellung einer Vielzahl von Makrozuständen,
- eine Erkennungseinheit (170) zur Erkennung eines Standardmakrozustands unter der Vielzahl von Makrozuständen als Makrozustand, der während der Stimulation die größte Suppression zeigt, wobei eine Suppression eines Makrozustand während eines sensorischen Stimulus als Differenz zwischen der Wahrscheinlichkeit des Auftretens des Makrozustands während des sensorischen Stimulus in einem Zeitraum vor dem sensorischen Stimulus bestimmt wird, und
- eine Bestimmungseinheit zur Bestimmung der auffälligen Reaktivität auf der Basis einer Differenz zwischen der Suppression des Standardmakrozustands während des sensorischen Zielstimulus und einer Suppression des Standardmakrozustands während des sensorischen Bezugsstimulus.

12. Ein computerlesbarer Programmcode zur Speicherung von Medien; der Programmcode umfasst Anweisungen zur Ausführung des Verfahrens (200) nach einem der Ansprüche 1 bis 10, wenn die Ausführung durch ein Gerät erfolgt, das in Anspruch 11 definiert ist.

## Revendications

1. Une méthode assistée par ordinateur (200) de détermination de l'importance de la réactivité d'au moins un stimulus sensoriel cible appliqué à un sujet, cette méthode comprenant :
- l'obtention (210) de signaux physiologiques de la part du sujet pendant une stimulation avec le stimulus sensoriel cible et pendant une stimulation avec un stimulus sensoriel de référence, les signaux physiologiques comprenant plusieurs canaux, et où les signaux physiologiques sont ceux d'un électroencéphalogramme (EEG),
- la détermination (220) à plusieurs points dans le temps des signaux physiologiques, des cartes de puissance spectrale topographiques, où une carte de puissance spectrale topographique comprend les puissances spectrales des bandes de fréquence des multiples canaux,
- le regroupement (230) des cartes de puissance spectrale topographiques pour obtenir plusieurs macro-états,
- l'identification (240) parmi ces multiples macro-états, d'un macro-état par défaut comme celui présentant la plus grande suppression pendant la stimulation, où une suppression d'un macro-état pendant un stimulus sensoriel est définie comme la différence entre la probabilité d'occurrence du macro-état pendant le stimulus sensoriel et à la probabilité d'occurrence du macro-état dans une période précédant le stimulus sensoriel, et
- la détermination (250) de m'importance de la réactivité en fonction de la différence entre la suppression du macro-état par défaut pendant le stimulus sensoriel cible et une suppression du macro-état par défaut pendant le stimulus sensoriel de référence.

2. La méthode (200) de la revendication 1, où pendant un essai de stimulation, le stimulus sensoriel cible est appliqué pendant une période de stimulation se trouvant après une première période sans stimulation et avant une deuxième période sans stimulation, où la durée de la première période et la durée de la seconde période sont au moins aussi longues que la durée de la période de stimulation.

3. La méthode (200) d'une des revendications précédentes, où les bandes de fréquence incluent les bandes de fréquence delta, thêta, alpha et bêta conventionnelles d'EEG.

4. La méthode (200) d'une des revendications précédentes, comprenant de plus une étape de détermination des multiples points dans le temps comme ceux correspondant aux extrêmes d'une variation dans les cartes de puissance spectrale topographiques.

5. La méthode (200) d'une des revendications précédentes, où le regroupement (230) des cartes de puissance spectrale topographiques comprend un regroupement hiérarchique à l'aide d'une mesure de distance cosinus.

6. La méthode (200) d'une des revendications précédentes, où la méthode est répétée sur plusieurs essais, et le regroupement effectué sur ces plusieurs essais.

7. La méthode (200) d'une des revendications précédentes, où le au moins un stimulus sensoriel cible comprend plusieurs stimuli.

8. La méthode (200) de la revendication 7, où les plusieurs stimuli sont appliqués alternativement.

9. La méthode (200) des revendications précédentes, comprenant de plus l'optimisation d'une moyenne de l'importance de la réactivité sur plusieurs essais d'un type de stimulus, pour définir un nombre optimal de classes de macro-états.

10. La méthode (200) d'une des revendications précédentes, comprenant de plus l'affichage du macro-état par défaut sous forme de séquence de cartes de puissance topographiques mises à l'échelle sur l'axe Z.

11. Un appareillage (100) de détermination de l'importance de la réactivité d'au moins un stimulus sensoriel cible appliqué à un sujet, l'appareillage comprenant :
- une unité d'obtention (110) permettant d'obtenir les signaux physiologiques de la part du sujet pendant une stimulation avec le stimulus sensoriel cible et pendant une stimulation avec un stimulus sensoriel de référence, les signaux physiologiques comprenant plusieurs canaux, où les signaux physiologiques sont des signaux d'EEG,
- une unité de détermination (170) pour déterminer, à plusieurs points dans le temps des signaux physiologiques, des cartes de puissance spectrale topographiques, où une carte de puissance spectrale topographique comprend les puissances spectrales des bandes de fréquence des multiples canaux,
- une unité de regroupement (170) pour le regroupement des cartes de puissance spectrale topographiques pour obtenir plusieurs macro-états,
- une unité d'identification (170) pour l'identification, parmi ces multiples macro-états, d'un macro-état par défaut comme celui présentant la plus grande suppression pendant la stimulation, où une suppression d'un macro-état pendant un stimulus sensoriel est définie comme la différence entre la probabilité d'occurrence du macro-état pendant le stimulus sensoriel et à la probabilité d'occurrence du macro-état dans une période précédant le stimulus sensoriel, et
- une unité de différence pour le calcul de l'importance de la réactivité en fonction de la différence entre la suppression du macro-état par défaut pendant le stimulus sensoriel cible et une suppression du macro-état par défaut pendant le stimulus sensoriel de référence.

12. Un moyen de stockage lisible par ordinateur pour l'enregistrement du code du programme, le code de programme comprenant des instructions qui lors de leur exécution par un appareil tel que défini dans la revendication 11, réalise la méthode (200) d'une des revendications 1 à 10.
